# EUROPÄISCHE PATENTSCHRIFT

(11) **EP 3 185 846 B1**
(45) Veröffentlichungstag und Bekanntmachung des Hinweises auf die Patenterteilung: **13.03.2019**
(21) Anmeldenummer: 15753953.7
(22) Anmeldetag: 25.08.2015
(51) Int. Cl.: A61K 8/365, A61K 8/42, A61K 8/46, A61Q 5/02, A61Q 5/12, A61K 8/73, A61K 8/19

(54) **KONDITIONIERENDES HAARREINIGUNGSMITTEL**
CONDITIONING HAIR-CLEANING AGENT
COMPOSITION NETTOYANTE CAPILLAIRE DE CONDITIONNEMENT

(30) Priorität: 29.08.2014 DE 102014217311
(43) Veröffentlichungstag der Anmeldung: 05.07.2017
(73) Patentinhaber: Henkel AG & Co. KGaA, 40589 Düsseldorf (DE)
(72) Erfinder: HENTRICH, Dirk, 22457 Hamburg (DE); BENDER, Corinna, 22457 Hamburg (DE)
(86) Internationale Anmeldenummer: PCT/EP2015/069395
(87) Internationale Veröffentlichungsnummer: WO 2016/030347

(56) Entgegenhaltungen:
- EP-A1- 0 937 449
- EP-A1- 1 479 369
- EP-A1- 2 255 776
- WO-A1-95/01152
- WO-A1-03/086335
- WO-A2-2012/038536
- DE-A1-102012 201 861
- DATABASE GNPD MINTEL; 30. September 2011 (2011-09-30), "Fresh Blast Shower Gel", XP002745752, Database accession no. 1621123

## Beschreibung

Die Erfindung liegt auf dem Gebiet der Kosmetik und betrifft Haarreinigungsmittel mit saurem pH-Wert, die ein anionisches Tensid, ein nichtionisches Tensid, ein kationisches Pflegepolymer sowie Calciumlactat oder ein Gemisch aus Milchsäure und einem anorganischen Calciumsalz enthalten. Die Erfindung betrifft weiterhin die Verwendung der Reinigungsmittel zur Verbesserung der Entwirrbarkeit und Kämmbarkeit insbesondere geschädigter Haare sowie zur Verminderung der elektrostatischen Aufladung von Haaren und des Auftretens struppiger oder unerwünscht gekräuselter Haare.

Bedingt durch Umwelteinflüsse (wie beispielsweise intensive Sonneneinstrahlung), mechanische Belastungen (wie beispielsweise intensives Kämmen unter Föhnhitze) physikalische oder chemische Behandlungen (wie beispielsweise Färben, Verformen oder Glätten der Haare) können Haare in ihrer Struktur geschädigt werden. Die Folgen sind oftmals Spliss, Haarbruch und/oder fehlender Haarglanz. Darüber hinaus lassen sich geschädigte Haare schlecht entwirren und frisieren.

Haarreinigungsmittel umfassen in der Regel (anionische) Tenside. Diese sind erwünscht und notwendig, damit Talg und andere Verschmutzungen von der Haaroberfläche und der Kopfhaut entfernt werden können. Sie sind aber auch ursächlich für die Entfernung von Lipiden und Proteinen aus den Haaren oder der Kopfhaut während der Reinigung, so dass eine nachfolgende Behandlung mit Pflegemitteln zur Regenerierung der Haare erforderlich ist.

Die Reinigung bereits geschädigter Haare ist daher problematisch und es besteht das Bedürfnis nach besonders milden Haarreinigungsmitteln, die geschädigte Haare während der Reinigung nicht zusätzlich belasten und sie pflegen.

Die Formulierung so genannter konditionierender Haarreinigungsmittel ist bekannt. Beispielsweise werden in EP 1329215 pflegende Haarreinigungsmittel vorgeschlagen, die bei 20-facher Verdünnung einen pH-Wert im Bereich von 1-4 aufweisen und ein anionisches Sulfattensid, ein kationisches Guar-Gum, eine Hydroxycarbonsäure (Glykolsäure, Milchsäure oder Äpfelsäure) und ein Salz (insbesondere anorganische oder organische Natriumsalze) enthalten. Die Haarreinigungsmittel weisen besonders gute Schaumeigenschaften auf und verleihen den damit gereinigten Haaren mehr Weichheit, Glanz und Geschmeidigkeit. EP 0937449 betrifft Haarreinigungsmittel enthaltend Gamma-Oryzanol und Calciumsalzen.

Diese Technik ist jedoch vor allem bei der Behandlung geschädigter Haare und/oder trockener Kopfhaut nicht zweckdienlich, denn die starke Acidität der Mittel kann zu einer zusätzlichen Austrockung der Kopfhaut und zu unerwünschter Schuppenbildung führen. Auch die Entwirrbarkeit insbesondere langer, geschädigter Haare war nach der Behandlung mit den Mitteln nicht zufriedenstellend.

Es besteht demnach weiterhin der Bedarf nach milden, die Kopfhaut schonenden Haarreinigungsmitteln, die sich insbesondere für die Reinigung geschädigter Haare eignen und diesen vorteilhafte optische und haptische Eigenschaften verleihen.

Der vorliegenden Anmeldung lag die Aufgabe zugrunde, milde, gut haut- und haarverträgliche, pflegende Haarreinigungsmittel bereitzustellen, die Haarschädigungen mildern.
Besonders lange, geschädigte Haare sollen während der Reinigung in ihrer Struktur gestärkt werden und sich nach der Reinigung leicht entwirren lassen.

Es wurde gefunden, dass die Hautverträglichkeit und Milde pflegender Haarreinigungsmittel signifikant gesteigert werden kann, wenn sie eine Mischung aus milden anionischen und nichtionischen Tensiden, kationischen Polymeren und einem speziellen Salz (oder einer speziellen Säure-Salz-Mischung) enthalten und einen leicht aciden pH-Wert aufweisen.

Ein erster Gegenstand der Erfindung ist daher ein Haarreinigungsmittel, das - bezogen auf sein Gesamtgewicht -
a) 6 bis 12 Gew.-% mindestens eines anionischen Tensids,
b) 0,1 bis 5 Gew.-% mindestens eines nichtionischen Tensids,
c) 0,01 bis 2 Gew.-% mindestens eines kationischen Pflegepolymeren und
d)
   (i) 0,01 bis 3 Gew.-% Calciumlactat oder
   (ii) 0,1 bis 5 Gew.-% Milchsäure und 0,05 bis 3 Gew.-% mindestens eines anorganischen Calciumsalzes enthält,
und einen pH-Wert im Bereich von 4,3 bis 5,0 aufweist.

Die erfindungsgemäßen Haarreinigungsmittel weisen eine Reihe von Vorteilen auf: sie sind besonders mild und belasten oder schädigen die (trockene) Kopfhaut nicht. Des Weiteren verleihen sie den damit behandelten Haaren in nassem wie in trockenem Zustand eine bessere Entwirrbarkeit und Kämmbarkeit, vermindern die elektrostatische Aufladung und das "Fliegen" von Haaren sowie das Auftreten struppiger oder unerwünscht gekräuselter Haare ("anti-frizz-Effekt").
Die erfindungsgemäßen Haarreinigungsmittel enthalten die Wirkstoffkombination a) - d) bevorzugt in einem kosmetischen akzeptablen Träger. Unter einem kosmetisch akzeptablen Träger wird im Rahmen der Erfindung bevorzugt ein wässriger oder wässrig-alkoholischer Träger verstanden. Besonders bevorzugt enthält der kosmetische Träger mindestens 50 Gew.-%, mehr bevorzugt mindestens 60 Gew.-% und besonders bevorzugt mindestens 65 Gew.-% Wasser.

Der kosmetische Träger kann weiterhin 0,01 bis 40 Gew.-%, bevorzugt 0,05 bis 30 Gew.-% und insbesondere 0,1 bis 20 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.
Besonders bevorzugt sind die wasserlöslichen Alkohole.
Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Besonders milde Haarreinigungsmittel mit hervorragenden Pflegeeigenschaften für geschädigte Haare konnten in einem engeren pH-Bereich hergestellt werden.

In einer ersten bevorzugten Ausführungsform weisen die erfindungsgemäßen Haarreinigungsmittel daher einen pH-Wert im Bereich von 4,4 bis 5,0, mehr bevorzugt von 4,5 bis 4,9 und insbesondere von 4,5 bis 4,8 auf.

Zu den geeigneten anionischen Tensiden, die in den erfindungsgemäßen Haarreinigungsmitteln eingesetzt werden können, zählen beispielsweise:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfate und/oder Alkylpolyglykolethersulfatsalze der Formel R-(O-CH₂-CH₂)ₙ-OSO₃X, in der R bevorzugt eine lineare oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 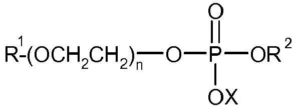 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Besonders bevorzugt im Rahmen der vorliegenden Erfindung sind milde, die Kopfhaut schonende anionische Tenside, worunter im Rahmen der vorliegenden Erfindung bevorzugt Ethercarbonsäuren, Acylisethionate, Sulfobernsteinsäureester und Alkyl(polyglykolether)sulfate zu verstehen sind.

In einer zweiten bevorzugten Ausführungsform enthalten die erfindungsgemäßen Haarreinigungsmittel daher als anionische Tenside bevorzugt Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist, Acylisethionate mit 8 bis 24 C-Atomen in der Acylgruppe, Sulfobernsteinsäuremono- und/oder -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen und/oder Alkylsulfat- und/oder Alkylpolyglykolethersulfatsalze der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X⁺ ein Alkali-, Erdalkali-, Ammonium- oder Alkanolaminion bedeutet.

Ganz besonders bevorzugt sind Alkylethersulfatsalze der Formel R-(OCH₂-CH₂)ₓ-OSO₃⁻ X⁺, in der R bevorzugt für einen geradkettigen oder verzweigten Alkylrest mit 8 bis 18 und insbesondere mit 10 bis 16 C-Atomen, x für 1 bis 6, insbesondere für 2 bis 4 Ethylenoxideinheiten, und X⁺ für ein Natrium-, Magnesium und/oder Triethanolaminion steht.
Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen, wie sie beispielsweise unter der INCI-Bezeichnung Sodium Laureth Sulfate oder Sodium Trideceth Sulfate im Handel erhältlich sind.
Diese Tenside weisen eine besonders ausgewogene Balance zwischen Schaumstärke und Milde auf.

Ein allzu hoher Gehalt an anionischen Tensiden ist für die erfindungsgemäßen milden Haarreinigungsmittel nicht geeignet, deshalb enthalten sie - bezogen auf ihr Gewicht - 6 bis 12 Gew.-% eines oder mehrerer anionischen(r) Tensids(e).
Insbesondere bevorzugt enthalten die erfindungsgemäßen Haarreinigungsmittel Alkylsulfat- und/oder Alkylpolyglykolethersulfatsalze der zuvor genannten Formel in Mengen von 6 bis 12 Gew.-%.

Eine weitere bevorzugte Ausführungsform der Erfindung ist demnach dadurch gekennzeichnet, dass die Haarreinigungsmittel - bezogen auf ihr Gesamtgewicht - 6 bis 12 Gew.-%, bevorzugt 7 bis 11 Gew.-% und insbesondere 8 bis 10 Gew.-% mindestens eines anionischen Alkylethersulfatsalzes der Formel R-(OCH₂-CH₂)ₓ-OSO₃⁻ X⁺ enthalten, in der R für einen geradkettigen oder verzweigten Alkylrest mit 8 bis 18 und insbesondere mit 10 bis 16 C-Atomen, x für 1 bis 6 und insbesondere für 2 bis 4 Ethylenoxideinheiten und X⁺ für ein Natrium-, Magnesium und/oder Triethanolaminion steht.

Zur Erzielung optimaler Schaumeigenschaften bei gleichzeitiger Milde enthalten die erfindungsgemäßen Haarreinigungsmittel neben mindestens einem anionischen Tensid - bezogen auf ihr Gesamtgewicht - 0,1 bis 5 Gew.-% mindestens eines nichtionischen Tensids.

Zu den erfindungsgemäß geeigneten nichtionischen Tensiden/Emulgatoren zählen bevorzugt
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Fettsäurealkanolamiden der allgemeinen Formel 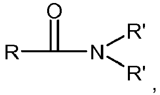 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Anlagerungsprodukte von Ethylenoxid an Fettamine und/oder
- Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside", "Decyl Glucoside" und/oder "Lauryl Glucoside" im Handel erhältlich sind

Die Verwendung schaumstarker und/oder schaumstabilisierender nichtionischer, milder Tenside hat sich als äußerst zufriedenstellend erwiesen, deshalb sind in einer dritten besonders bevorzugten Ausführungsform erfindungsgemäße Haarreinigungsmittel dadurch gekennzeichnet, dass sie mindestens ein nichtionisches Tensid, ausgewählt aus C₈-C₃₀-Fettsäuremono- und/oder -diestern von Anlagerungsprodukten von 1 bis 30 Mol Ethylenoxid an Glycerin, Anlagerungsprodukten von 2 bis 50 Mol Ethylenoxid und/oder 0 bis 5 Mol Propylenoxid an lineare und verzweigte Fettalkohole mit 8 bis 30 C-Atomen, Fettsäurealkanolamiden der allgemeinen Formel 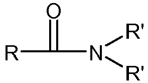 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht, und/oder Alkylpolyglucosiden enthalten.

Neben der Milde weisen Fettsäurealkanolamide schaumstabilisierende, verdickende und pflegende (rückfettende) Eigenschaften auf, deshalb sind in einer weiteren vorteilhaften Ausführungsform erfindungsgemäße Haarreinigungsmittel besonders bevorzugt, die - bezogen auf ihr Gesamtgewicht - 0,1 bis 5 Gew.-%, bevorzugt 0,2 bis 3 Gew.-% und insbesondere 0,4 bis 2 Gew.-% mindestens eines Fettsäurealkanolamids der zuvor genannten Formel enthalten. Insbesondere bevorzugt ist ein unter der INCI-Bezeichnung Cocamide MEA bekanntes Fettsäurealkanolamid.

Neben der Tensidmischung aus anionischen und nichtionischen Tensiden in spezifischen Mengen enthalten die erfindungsgemäßen Haarreinigungsmittel zur Erhöhung der Pflegeeigenschaften geschädigter Haare mindestens ein kationisches Pflegepolymer in einer Menge von 0,1 bis 2 Gew.-% (bezogen auf das Gesamtgewicht der Haarreinigungsmittel).

Unter geeigneten kationischen Pflegepolymeren sind beispielsweise zu verstehen:
- quaternisierte Cellulosepolymere, vor allem Polyquaternium-10, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar N-Hance® und Jaguar® vertriebenen Produkte,
- polymere Dimethyldiallylammoniumsalze und deren Copolymere mit Estern und Amiden von Acrylsäure und Methacrylsäure, vor allem Polyquaternium-6 und Polyquaternium-7. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Besonders bevorzugte kationische Polymere sind quaternisierte Cellulosepolymere, hydrophob modifizierte quaternisierte Cellulosepolymere, kationische Guarderivate und/oder kationische Polymere auf Acrylsäure(derivat)basis, die besonders bevorzugt ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67.

In einer vierten bevorzugten Ausführungsform umfassen erfindungsgemäße Haarreinigungsmittel als kationisches Pflegepolymer daher mindestens ein quaternisiertes Cellulosepolymer, ein hydrophob modifiziertes quaternisiertes Cellulosepolymer, ein kationisches Guar-Derivat, ein polymeres Dimethyldiallylammoniumsalz und deren Copolymere mit Estern und/oder Amiden von Acrylsäure und Methacrylsäure, ein Copolymeres des Vinylpyrrolidons mit quaternierten Derivaten des Dialkylaminoalkylacrylats und -methacrylats und/oder Polyvinylpyrrolidon.

Als ganz besonders bevorzugte kationische Pflegepolymere für die erfindungsgemäßen Haarreinigungsmittel haben sich kationische Polysaccharidpolymere - insbesondere quaternisierte Cellulosepolymere herausgestellt.

In einer weiteren bevorzugten Ausführungsform enthalten erfindungsgemäße Haarreinigungsmittel - bezogen auf ihr Gesamtgewicht - daher 0,01 bis 2 Gew.-%, bevorzugt 0,1 bis 1,5 Gew.-% und insbesondere 0,2 bis 1 Gew.-% mindestens eines quaternisierten Cellulosepolymeren, insbesondere eines durch die Umsetzung von Hydroxyethylcellulose mit Trimethylammonium-substituierten Epoxiden erhältlichen und unter der INCI-Bezeichnung Polyquaternium-10 bekannten kationischen Polymeren.

Erst durch die Zugabe von Calciumlactat (oder alternativ von einer Mischung aus Milchsäure und einem anorganischen Calciumsalz) zu der Mischung aus anionischen Tensiden, nichtionischen Tensiden und kationischen Pflegepolymeren einerseits, sowie durch die Einstellung des pH-Wertes auf einen Wert im Bereich von 4,3 bis 5,0 andererseits, konnte die zuvor beschriebene, hervorragende Balance zwischen reinigenden, milden und pflegenden Eigenschaften der erfindungsgemäßen Haarreinigungsmittel erzielt werden.
Dabei ist es von Vorteil, wenn zur pH-Wert-Einstellung keine weiteren Säuren als Milchsäure und/oder Calciumlactat verwendet werden.

Für die Erzielung der genannten Pflegeeffekte spielt es keine Rolle, ob Calciumlactat oder eine Mischung aus Milchsäure und einem anorganischen Calciumsalz in den Haarreinigungsmitteln eingesetzt wird.
Bei der Auswahl der anorganischen Calciumsalze haben sich jedoch Calciumchlorid, Calciumhydroxid, Calciumsulfat und/oder Calciumoxid als besonders geeignet erwiesen.

Ebenso wurde gefunden, dass Calciumlactat oder die Mischung aus Milchsäure und einem anorganischen Calciumsalz in einer bestimmten Menge in den Haarreinigungsmitteln eingesetzt werden muss, um optimale Pflegeeffekte zu erzielen.
So konnten mit Haarreinigungsmitteln, die - bezogen auf ihr Gesamtgewicht -
i. 0,01 bis 3 Gew.-%, bevorzugt 0,02 bis 2,5 Gew.-% und insbesondere 0,05 bis 2 Gew.-% Calciumlactat oder
ii. 0,1 bis 5 Gew.-%, bevorzugt 0,5 bis 4 Gew.-% und insbesondere 1 bis 3 Gew.-% Milchsäure sowie 0,05 bis 3 Gew.-%, bevorzugt 0,1 bis 2 Gew.-% uns insbesondere 0,2 bis 1 Gew.-% Calciumhydroxid enthalten, besonders gute Pflegeeffekte (Entwirrbarkeit, Kämmbarkeit und anti-frizz-Effekt) erzielt werden.

In einer fünften bevorzugten Ausführungsform enthalten erfindungsgemäße Haarreinigungsmittel als anorganisches Calciumsalz d) daher Calciumchlorid, Calciumhydroxid, Calciumsulfat und/oder Calciumoxid.

In einer sechsten bevorzugten Ausführungsform enthalten erfindungsgemäße Haarreinigungsmittel - bezogen auf ihr Gesamtgewicht - daher
i. 0,01 bis 3 Gew.-%, bevorzugt 0,02 bis 2,5 Gew.-% und insbesondere 0,05 bis 2 Gew.-% Calciumlactat oder
ii. 0,1 bis 5 Gew.-%, bevorzugt 0,5 bis 4 Gew.-% und insbesondere 1 bis 3 Gew.-% Milchsäure sowie 0,05 bis 3 Gew.-%, bevorzugt 0,1 bis 2 Gew.-% uns insbesondere 0,2 bis 1 Gew.-% Calciumhydroxid.

Eine ganz besonders bevorzugte Ausführungsform der vorliegenden Erfindung ist dadurch gekennzeichnet, dass die Haarreinigungsmittel - bezogen auf ihr Gesamtgewicht -
a) 6 bis 12 Gew.-% mindestens eines anionischen Alkylethersulfatsalzes der Formel R-(OCH₂-CH₂)ₓ-OSO₃⁻ X⁺, in der R für einen geradkettigen oder verzweigten Alkylrest mit 8 bis 18 und insbesondere mit 10 bis 16 C-Atomen, x für 1 bis 6 und insbesondere für 2 bis 4 Ethylenoxideinheiten und X⁺ für ein Natrium-, Magnesium und/oder Triethanolaminkation steht,
b) 0,1 bis 5 Gew.-% mindestens eines Fettsäurealkanolamids der Formel 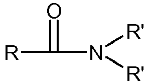 in der R bevorzugt einen linearen oder verzweigten, gesättigten oder ungesättigten Alkyl-oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
c) 0,01 bis 2 Gew.-% mindestens eines quaternisierten Cellulosepolymeren, und
d)
   (i) 0,01 bis 3 Gew.-% Calciumlactat oder
   (ii) 0,1 bis 5 Gew.-% Milchsäure sowie 0,05 bis 3 Gew.-% Calciumhydroxid enthalten, und einen pH-Wert im Bereich von 4,3 bis 5,0, bevorzugt von 4,4 bis 5,0, bevorzugt von 4,5 bis 4,9 und insbesondere von 4,5 bis 4,8 aufweisen.

Innerhalb dieser Ausführungsform sind Haarreinigungsmittel insbesondere bevorzugt, die - bezogen auf ihr Gesamtgewicht -
a) 1 bis 11 Gew.-% mindestens eines Natrium-, Magnesium und/oder Triethanolaminsalzes eines linearen oder verzweigten Lauryl-, Tridecyl- und/oder Myristylsulfats, das einen Ethoxylierungsgrad von 2 bis 4 aufweist,
b) 0,2 bis 3 Gew.-% mindestens eines unter der INCI-Bezeichnung Cocamide MEA bekannten Fettsäurealkanolamids,
c) 0,1 bis 1,5 Gew.-% mindestens eines unter der INCI-Bezeichnung Polyquaternium-10 bekannten quaternisierten Cellulosepolymeren, und
d)
   (i) 0,02 bis 2,5 Gew.-% Calciumlactat oder
   (ii) 0,5 bis 4 Gew.-% Milchsäure sowie 0,1 bis 2 Gew.-% Calciumhydroxid enthalten,
und einen pH-Wert im Bereich von 4,5 bis 4,9 und insbesondere von 4,5 bis 4,8 aufweisen.

Die optischen und haptischen Eigenschaften der erfindungsgemäßen Haarreinigungsmittel können noch weiter gesteigert werden, wenn Ihnen weitere Haarpflegestoffe hinzugefügt werden.
Als geeignete weitere Haarpflegestoffe kommen bevorzugt kosmetisch geeignete Öl-, Fett- und/oder Wachskomponenten, Vitamine und/oder Proteinhydrolysate in Betracht.

Kosmetisch geeignete Öl-, Wachs- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).

Als Ölkomponente kann weiterhin ein Dialkylether dienen.
Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.
Besonders bevorzugt ist der Di-n-octylether, der im Handel unter der Bezeichnung Cetiol® OE erhältlich ist.

Als synthetische Öle kommen bevorzugt Silikonverbindungen in Betracht.
Silikone bewirken auf dem Haar ausgezeichnete konditionierende Eigenschaften. Insbesondere wirken sie sich in vielen Fällen positiv auf den Haargriff und die Weichheit der Haare aus.
Es ist daher erstrebenswert, in kosmetischen Haarbehandlungsmitteln Silikone einzusetzen. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Unter Fettstoffen sind zu verstehen Fettsäuren, Fettalkohole sowie natürliche und synthetische Wachse, welche sowohl in fester Form als auch flüssig in wässriger Dispersion vorliegen können. Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

### Weitere Fettstoffe sind beispielsweise

- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren
   technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls® 90-O18, Monomuls® 90-L12, Cetiol® HE oder Cutina® MD.

Der Gewichtsanteil der Öl-, Wachs- und/oder Fettkomponenten am Gesamtgewicht der erfindungsgemäßen Haarreinigungsmittel beträgt bevorzugt 0,01 bis 5 Gew.-%, besonders bevorzugt 0,025 bis 4 Gew.-% und insbesondere 0,05 bis 3 Gew.-%.

Für die Erhöhung des Haarglanzes und zur Erzielung eines weicheren Haargriffs hat es sich als besonders bevorzugt erwiesen, wenn die erfindungsgemäßen Haarreinigungsmittel als Öl-, Fett- und/oder Wachskomponente weiterhin mindestens ein Silikon und/oder mindestens ein pflanzliches Öl enthalten.

Eine weitere bevorzugte Ausführungsform der Erfindung ist demnach dadurch gekennzeichnet, dass sie - bezogen auf ihr Gesamtgewicht - zusätzlich 0,1 bis 5 Gew.-% mindestens eines Silikons und/oder mindestens eines pflanzlichen Öls enthält.

Unter geeigneten Proteinhydrolysaten sind Produktgemische zu verstehen, die durch sauer, basisch oder enzymatisch katalysierten Abbau von Proteinen (Eiweißen) erhalten werden können. Es können Proteinhydrolysate pflanzlichen, tierischen und/oder marinen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Der Gewichtsanteil des oder der Proteinhydrolysats(e) am Gesamtgewicht der erfindungsgemäßen Haarreinigungsmittel beträgt bevorzugt 0,001 bis 5 Gew.-%, mehr bevorzugt 0,025 bis 3 Gew.-% und insbesondere 0,05 bis 2 Gew.-%.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   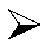 Vitamin B₁ (Thiamin)
   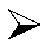 Vitamin B₂ (Riboflavin)
   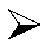 Vitamin B₃. Unter dieser Bezeichnung werden häufig die Verbindungen Nicotinsäure und Nicotinsäureamid (Niacinamid) geführt.
   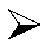 Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   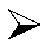 Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Ganz besonders bevorzugt sind Nicotinsäureamid, Biotin, Pantolacton und/oder Panthenol. Insbesondere bevorzugt ist Panthenol.

Der Gewichtsanteil des oder der Vitamins(e), Vitaminderivats(e), und/oder der Vitaminvorstufe(n) am Gesamtgewicht der kosmetischen Mittel beträgt bevorzugt 0,001 bis 2 Gew.-%, besonders bevorzugt 0,005 bis 1 Gew.-% und insbesondere 0,01 bis 0,5 Gew.-%.

Eine weitere bevorzugte Ausführungsform der Erfindung ist dadurch gekennzeichnet, dass sie - bezogen auf ihr Gesamtgewicht - zusätzlich 0,001 bis 2 Gew.-% mindestens eines Vitamins, Vitaminderivats und/oder Vitaminsalzes und/oder 0,001 bis 5 Gew.-% mindestens eines - gegebenenfalls kationisierten - Proteinhydrolysats enthält.

Es kann weiterhin von Vorteil sein, den erfindungsgemäßen Haarreinigungsmitteln weiterhin einen UV-Filter hinzuzufügen, um insbesondere bereits geschädigte Haare vor schädlicher UV-Strahleneinwirkung zu schützen.

In einer weiteren bevorzugten Ausführungsform enthalten erfindungsgemäße Haarreinigungsmittel (bezogen auf ihr Gesamtgewicht) daher weiterhin 0,01 bis 0,5 Gew.-% mindestens eines UV-Filters.

Geeignete UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA- (315-400 nm), im UVB- (280-315nm) oder im UVC-(<280 nm) Bereich liegt. UV-Filter mit einem Absorptionsmaximum im UVB-Bereich, insbesondere im Bereich von etwa 280 bis etwa 300 nm, sind besonders bevorzugt.

Die UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Für die Herstellung uns Stabilisierung der erfindungsgemäßen Haarreinigungsmittel sind besonders wasserlösliche UV-Filter geeignet, wie beispielsweise der unter der INCI-Bezeichnung Benzophenone-4 bekannte UV-Filter.

Neben den zuvor genannten Wirk- und Trägerstoffen können die erfindungsgemäßen Haarreinigungsmittel noch eine Reihe weiterer Inhaltsstoffe enthalten, die ihnen vorteilhafte Eigenschaften verleihen. Zu den bevorzugten fakultativen Wirkstoffen, die in den erfindungsgemäßen Mitteln eingesetzt werden können, zählen beispielsweise:
- amphotere und/oder zwitterionische Tenside, die in den Haarreinigungsmitteln (bezogen auf deren Gesamtgewicht) bevorzugt in einer Menge von 0,5 bis 20 Gew.-%, mehr bevorzugt von 1 bis 15 Gew.-%, besonders bevorzugt von 1,25 bis 10 Gew.-% und insbesondere von 1,5 bis 7,5 Gew.-% eingesetzt werden können, und
- Antischuppenwirkstoffe, die in den in den Haarreinigungsmitteln (bezogen auf deren Gesamtgewicht) bevorzugt in einer Menge von 0,01 bis 7,5 Gew.-%, besonders bevorzugt von 0,05 bis 5 Gew.-% und insbesondere von 0,1 bis 3 Gew.-% eingesetzt werden können.

Zu den geeigneten amphoteren und/oder zwitterionischen Tensiden die in den erfindungsgemäßen Haarreinigungsmitteln eingesetzt werden können, zählen beispielsweise eine oder mehrere Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

| | |
|---|---|
| (I) | (II) |
| 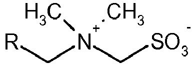 | 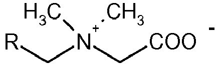 |
| (III ) | (IV) |
| 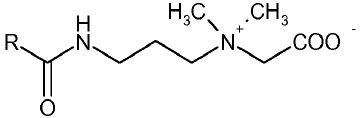 | 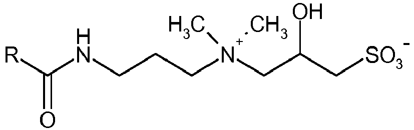 |
| (V) | (VI) |
| 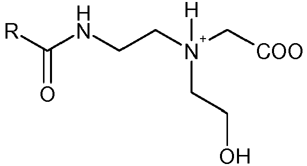 | 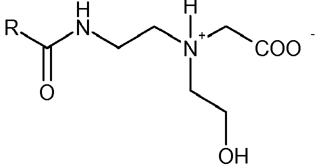 |
| (VII) | |
| 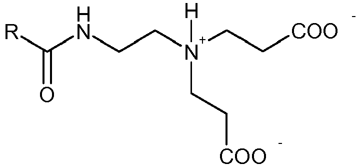 | |

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 18 und insbesondere mit 8 bis 16 C-Atomen.
Besonders bevorzugt sind amphotere und/oder zwitterionische Tenside, bei denen sich der Rest R von Kokosfett ableitet.
Ganz besonders bevorzugt sind die unter den INCI-Bezeichnungen Sodium Cocoamphoacetate, Disodium Cocoamphodiacetate, Sodium Lauroamphoacetate, Sodium Lauroamphodiacetate, Sodium Cocoamphopropionate, Disodium Cocoamphodipropionate, Coco Betaine, Lauryl Betaine Cocamidopropylbetain und/oder Lauramidopropylbetain bekannten und im Handel von mehreren Anbietern erhältlichen amphoteren/zwitterionischen Tenside.
Insbesondere bevorzugt sind Tenside mit den INCI-Bezeichnungen Cocoampho(di)acetate und/oder Cocamidopropylbetain.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Besonders bevorzugt sind Climbazol, Zink Pyrithion und Piroctone Olamine, insbesondere bevorzugt ist Zink Pyrithion.

Zu den weiteren fakultativen Komponenten, die in den erfindungsgemäßen Haarreinigungsmitteln eingesetzt werden können, zählen beispielsweise
- Pflanzenextrakte und/oder
- Feuchthaltemittel.

Unter geeigneten Pflanzenextrakten sind Extrakte zu verstehen, die aus allen Teilen einer Pflanze hergestellt werden können. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.
Besonders bevorzugt für die Verwendung in den erfindungsgemäßen Zusammensetzungen sind die Extrakte aus Grünem Tee, Brennessel, Hamamelis, Kamille, Aloe Vera, Ginseng, Echinacea purpurea, Olea europea und/oder Boerhavia Diffusa-Wurzeln.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Die Pflanzenextrakte können in den erfindungsgemäßen Haarreinigungsmitteln (bezogen auf das Gesamtgewicht der Mittel) bevorzugt in einer Menge von 0,01 bis 10 Gew.-%, mehr bevorzugt von 0,05 bis 7,5 Gew.-% und insbesondere von 0,1 bis 5 Gew.-% eingesetzt werden.

Geeignete Feuchthaltemittel bzw. Penetrationshilfsstoffe und/ oder Quellmittel, die den erfindungsgemäßen Haarreinigungsmitteln zugesetzt werden können, sind beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.
Die Feuchthaltemittel können in den erfindungsgemäßen Haarreinigungsmitteln - bezogen auf deren Gesamtgewicht - bevorzugt in Mengen von 0,01 bis 10 Gew.-%, mehr bevorzugt in Mengen von 0,05 bis 5 Gew.-% und insbesondere in Mengen von 0,1 bis 3 Gew.-% eingesetzt werden.

Weitere Wirk-, Hilfs- und Zusatzstoffe, die in den erfindungsgemäßen Haarreinigungsmitteln eingesetzt werden können, sind beispielsweise:
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Farbstoffe zum Anfärben des Mittels,
- Wirkstoffe wie Bisabolol,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Perlglanzmittel wie EGDS oder PEG-3 Distearate,
- Konservierungsmittel, wie beispielsweise Natriumbenzoat oder Salicylsäure,
- Viskositätsregler wie Salze (NaCl).

Ein zweiter Gegenstand der vorliegenden Erfindung ist die kosmetische Verwendung des erfindungsgemäßen Haarreinigungsmittels nach einem der Ansprüche 1 bis 14 zur Verbesserung der
Entwirrbarkeit und Kämmbarkeit insbesondere geschädigter Haare sowie zur Verminderung der Elektrostatischen Aufladung ("Fliegens") von Haaren und des Auftretens struppiger oder unerwünscht
gekräuselter Haare ("anti-frizz-Effekt").

Bezüglich bevorzugter Ausführungsformen der erfindungsgemäßen Verwendung gilt mutatis mutandis das zu dem erfindungsgemäßen Mittel Gesagte.

### Beispiele:

### 1) Herstellung erfindungsgemäßer Shampoos (Mengen in [Gew.-%1]:

| | **Bsp. 1** | **Bsp. 2** | **Bsp. 3** | **Bsp. 4** | **Bsp. 5** |
|---|---|---|---|---|---|
| **Sodium Laureth Sulfate (AS)** | 9,0 | 9,5 | 8 | 9 | 10 |
| **Disodium Cocoamphodiacetate (AS)** | 0,8 | | 1 | 0,5 | 0,5 |
| **Cocoamidopropyl Betaine (AS)** | 1,6 | 2,5 | 2 | 1,5 | 1,5 |
| **Cocamide MEA** | 0,9 | 0,5 | 0,6 | 0, 5 | 0,7 |
| **PEG-7 Glyceryl Cocoate** | 0,4 | 0,6 | 0,5 | | 0,7 |
| **PEG-40 Hydrogenated Castor Oil** | 0,3 | 0,3 | 0,3 | 0,4 | 0,4 |
| **Polyquaternium-10** | 0,5 | 0,2 | 0,4 | 0,1 | 0,3 |
| **Guar Hydroxypropyltrimonium Chloride** | | 0,3 | | 0,2 | |
| **PEG-12 Dimethicone** | 1,0 | 0,8 | | 0,7 | 1,0 |
| **Panthenol** | 0,2 | 0,2 | 0,1 | 0,2 | |
| **Niacinamid** | | | 0,1 | | 0,1 |
| **Marulaöl** | | 0,05 | | | 0,05 |
| **Jojobaöl** | | | 0,1 | | |
| **Aprikosenkernöl** | 0,1 | | | 0,1 | |
| **Benzophenone-4** | 0,1 | 0,1 | 0,1 | 0,1 | 0,1 |
| **Hydrogenated Castor Oil** | 0,1 | 0,15 | 0,2 | 0,1 | 0,1 |
| **Cocodimonium Hydroxypropyl Hydrolyzed Keratin** | 0,05 | 0,05 | 0,05 | | 0,05 |
| **Natriumbenzoat** | 0,5 | 0,5 | 0,5 | 0,5 | 0,5 |
| **Lactic Acid 80% DAB** | 2,4 | 2,5 | 2,2 | 2,6 | 2,4 |
| **Calcium Hydroxide** | 0,55 | 0,6 | 0,5 | 0,65 | 0,55 |
| **Parfum** | 0,5-1 | 0,5-1 | 0,5-1 | 0,5-1 | 0,5-1 |
| **Wasser** | ad 100 | ad 100 | ad 100 | ad 100 | ad 100 |
| **pH-Wert** | 4,5-4,75 (±0,2) | 4,5-4,75 (±0,2) | 4,5-4,75 (±0,2) | 4,5-4,75 (±0,2) | 4,5-4,75 (±0,2) |

### 2) Beurteilung erfindungsgemäßer Shampoos:

In einem Halbseitenversuch wurde eine Hälfte geschädigter, langer Haare mit einem erfindungsgemäßen Shampoo (FDH14CB0003\13), das dem o.g. Beispiel 1 entspricht, in üblicher Art gewaschen. Die andere Hälfte wurde mit der gleichen Menge eines herkömmlichen Pflegeshampoos (FDH14CB0003\12) in üblicher Art gewaschen. Das herkömmliche Pflegeshampoo entsprach in seiner Zusammensetzung ebenfalls dem o.g. Beispiel 1, außer dass zur Ansäuerung des Shampoos Zitronensäure (anstatt Milchsäure) verwendet wurde, und dass das Shampoo kein Calciumsalz enthielt. Beide Shampoos wiesen einen pH-Wert von 4,75 auf.

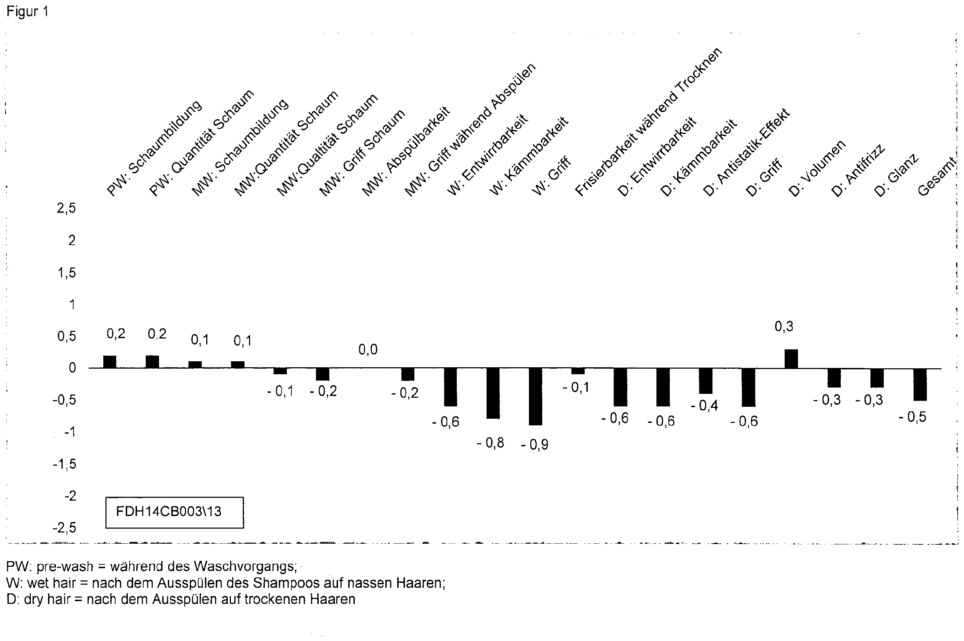

Nach dem Ausspülen der Shampoos mit Wasser wurden die u.g. Eigenschaften der nassen und trockenen Haare gleichzeitig und gegeneinander von 10 Experten auf einer Skala von 0 bis 2,5 bewertet (Unterschiede > 0,5 sind signifikant; diejenige Rezeptur, zu deren Seite der Ausschlag in dem Diagramm ist, wurde in der jeweiligen Eigenschaft von den Experten besser bewertet):
Dies verdeutlicht die Fig. 1 → auf der separaten Seite 26.

Aus dem Diagramm kann man deutlich erkennen, dass die Hälfte der Haare, die mit dem erfindungsgemäßen Shampoo gewaschen wurde, erkennbar bessere Pflegeeigenschaften im nassen und trockenen Zustand aufweist.

## Patentansprüche

1. Haarreinigungsmittel, enthaltend (bezogen auf sein Gesamtgewicht)
a) 6 bis 12 Gew.-% mindestens eines anionischen Tensids,
b) 0,1 bis 5 Gew.-% mindestens eines nichtionischen Tensids,
c) 0,01 bis 2 Gew.-% mindestens eines kationischen Pflegepolymeren und
d)
(i) 0,01 bis 3 Gew.-% Calciumlactat oder
(ii) 0,1 bis 5 Gew.-% Milchsäure und 0,05 bis 3 Gew.-% mindestens eines anorganischen Calciumsalzes,
**dadurch gekennzeichnet, dass** das Haarreinigungsmittel einen pH-Wert im Bereich von 4,3 bis 5,0 aufweist.

2. Haarreinigungsmittel nach Anspruch 1, **dadurch gekennzeichnet, dass** es einen pH-Wert im Bereich von 4,4 bis 5,0, bevorzugt von 4,5 bis 4,9 und insbesondere von 4,5 bis 4,8 aufweist.

3. Haarreinigungsmittel nach einem der vorhergehenden Ansprüche, **dadurch gekennzeichnet, dass** das anionische Tensid ausgewählt ist aus Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist, Acylisethionaten mit 8 bis 24 C-Atomen in der Acylgruppe, Sulfobernsteinsäuremono- und/oder -dialkylestern mit 8 bis 24 C-Atomen in der Alkylgruppe und Sulfobernsteinsäuremonoalkylpolyoxyethylestern mit 8 bis 24 C-Atomen in der Alkylgruppe und 1 bis 6 Oxyethylgruppen sowie aus Alkylsulfat- und/oder Alkylpolyglykolethersulfatsalzen der Formel R-(OCH₂-CH₂)ₓ-OSO₃⁻ X⁺, in der R bevorzugt eine lineare oder verzweigte, gesättigte oder ungesättigte Alkylgruppe mit 8 bis 30 C-Atomen, x die Zahl 0 oder 1 bis 12 und X⁺ ein Alkali-, Erdalkali-, Ammonium- oder Alkanolaminion bedeutet.

4. Haarreinigungsmittel nach einem der vorhergehenden Ansprüche, **dadurch gekennzeichnet, dass** es (bezogen auf sein Gesamtgewicht) 6 bis 12 Gew.-%, bevorzugt 7 bis 11 Gew.-% und insbesondere 8 bis 10 Gew.-% mindestens eines anionischen Alkylethersulfatsalzes der Formel R-(OCH₂-CH₂)ₓ-OSO₃⁻ X⁺ enthält, in der R für einen geradkettigen oder verzweigten Alkylrest mit 8 bis 18 und insbesondere mit 10 bis 16 C-Atomen, x für 1 bis 6 und insbesondere für 2 bis 4 Ethylenoxideinheiten und X⁺ für ein Natrium-, Magnesium und/oder Triethanolaminion steht.

5. Haarreinigungsmittel nach einem der vorhergehenden Ansprüche, **dadurch gekennzeichnet, dass** das nichtionische Tensid ausgewählt ist ausgewählt ist aus C₈-C₃₀-Fettsäuremono- und/oder
- diestern von Anlagerungsprodukten von 1 bis 30 Mol Ethylenoxid an Glycerin, Anlagerungsprodukten von 2 bis 50 Mol Ethylenoxid und/oder 0 bis 5 Mol Propylenoxid an lineare und verzweigte Fettalkohole mit 8 bis 30 C-Atomen, Fettsäurealkanolamiden der allgemeinen Formel 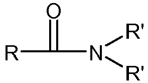 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht, und/oder Alkylpolyglucosiden.

6. Haarreinigungsmittel nach einem der vorhergehenden Ansprüche, **dadurch gekennzeichnet, dass** es (bezogen auf sein Gesamtgewicht) 0,1 bis 5 Gew.-%, bevorzugt 0,2 bis 3 Gew.-% und insbesondere 0,4 bis 2 Gew.-% mindestens eines Fettsäurealkanolamids der zuvor genannten Formel enthält, bevorzugt ein unter der INCI-Bezeichnung Cocamide MEA bekanntes Fettsäurealkanolamid.

7. Haarreinigungsmittel nach einem der vorhergehenden Ansprüche, **dadurch gekennzeichnet, dass** das kationische Pflegepolymer ausgewählt ist aus quaternisierten Cellulosepolymeren, hydrophob modifizierten quaternisierten Cellulosepolymeren, kationischen Guar-Derivaten, polymeren Dimethyldiallylammoniumsalzen und deren Copolymeren mit Estern und/oder Amiden von Acrylsäure und Methacrylsäure, Copolymeren des Vinylpyrrolidons mit quaternierten Derivaten des Dialkylaminoalkylacrylats und -methacrylats und/oder Polyvinylpyrrolidon.

8. Haarreinigungsmittel nach einem der vorhergehenden Ansprüche, **dadurch gekennzeichnet, dass** es (bezogen auf sein Gesamtgewicht) 0,01 bis 2 Gew.-%, bevorzugt 0,1 bis 1,5 Gew.-% und insbesondere 0,2 bis 1 Gew.-% mindestens eines quaternisierten Cellulosepolymeren, insbesondere eines durch die Umsetzung von Hydroxyethylcellulose mit Trimethylammonium-substituierten Epoxiden erhältlichen und unter der INCI-Bezeichnung Polyquaternium-10 bekannten kationischen Polymeren, enthält.

9. Haarreinigungsmittel nach einem der vorhergehenden Ansprüche, **dadurch gekennzeichnet, dass** es als anorganisches Calciumsalz d) (ii) Calciumchlorid, Calciumhydroxid, Calciumsulfat und/oder Calciumoxid enthält.

10. Haarreinigungsmittel nach einem der vorhergehenden Ansprüche, **dadurch gekennzeichnet, dass** es (bezogen auf sein Gesamtgewicht)
i. 0,01 bis 3 Gew.-%, bevorzugt 0,02 bis 2,5 Gew.-% und insbesondere 0,05 bis 2 Gew.-% Calciumlactat oder
ii. 0,1 bis 5 Gew.-%, bevorzugt 0,5 bis 4 Gew.-% und insbesondere 1 bis 3 Gew.-% Milchsäure sowie 0,05 bis 3 Gew.-%, bevorzugt 0,1 bis 2 Gew.-% uns insbesondere 0,2 bis 1 Gew.-% Calciumhydroxid enthält.

11. Haarreinigungsmittel nach einem der vorhergehenden Ansprüche, enthaltend (bezogen auf sein Gesamtgewicht)
a) 6 bis 12 Gew.-% mindestens eines anionischen Alkylethersulfatsalzes der Formel R-(OCH₂-CH₂)ₓ-OSO₃⁻ X⁺, in der R für einen geradkettigen oder verzweigten Alkylrest mit 8 bis 18 und insbesondere mit 10 bis 16 C-Atomen, x für 1 bis 6 und insbesondere für 2 bis 4 Ethylenoxideinheiten und X⁺ für ein Natrium-, Magnesium und/oder Triethanolaminkation steht,
b) 0,1 bis 5 Gew.-% mindestens eines Fettsäurealkanolamids der Formel 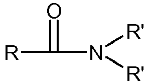 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
c) 0,01 bis 2 Gew.-% mindestens eines quaternisierten Cellulosepolymeren und
d)
(i) 0,01 bis 3 Gew.-% Calciumlactat oder
(ii) 0,1 bis 5 Gew.-% Milchsäure sowie 0,05 bis 3 Gew.-% Calciumhydroxid,
**dadurch gekennzeichnet, dass** das Haarreinigungsmittel einen pH-Wert im Bereich von 4,3 bis 5,0, bevorzugt von 4,4 bis 5,0, bevorzugt von 4,5 bis 4,9 und insbesondere von 4,5 bis 4,8 aufweist.

12. Haarreinigungsmittel nach einem der vorhergehenden Ansprüchen, **dadurch gekennzeichnet, dass** es (bezogen auf sein Gesamtgewicht) zusätzlich 0,01 bis 5 Gew.-% mindestens eines Silikons und/oder mindestens eines pflanzlichen Öls enthält.

13. Haarreinigungsmittel nach einem der vorhergehenden Ansprüche, **dadurch gekennzeichnet, dass** es (bezogen auf sein Gesamtgewicht) zusätzlich 0,001 bis 2 Gew.-% mindestens eines Vitamins, Vitaminderivats und/oder Vitaminsalzes und/oder 0,001 bis 5 Gew.-% mindestens eines - gegebenenfalls kationisierten - Proteinhydrolysats enthält.

14. Haarreinigungsmittel nach einem der vorhergehenden Ansprüche, **dadurch gekennzeichnet, dass** es (bezogen auf sein Gesamtgewicht) zusätzlich 0,01 bis 0,5 Gew.-% mindestens eines UV-Filters, bevorzugt eines wasserlöslichen UV-Filters, enthält.

15. Kosmetische Verwendung eines Haarreinigungsmittels nach einem der Ansprüche 1 bis 14 zur Verbesserung der Entwirrbarkeit und Kämmbarkeit insbesondere geschädigter Haare sowie zur Verminderung der elektrostatischen Aufladung ("Fliegens") von Haaren und des Auftretens struppiger oder unerwünscht gekräuselter Haare ("anti-frizz-Effekt").

## Claims

1. A hair cleansing agent containing (based on the total weight thereof)
a) 6 to 12 wt.% of at least one anionic surfactant,
b) 0.1 to 5 wt.% of at least one non-ionic surfactant,
c) 0.01 to 2 wt.% of at least one cationic conditioning polymer and
d)
(i) 0.01 to 3 wt.% calcium lactate or
(ii) 0.1 to 5 wt.% lactic acid and 0.05 to 3 wt.% of at least one inorganic calcium salt, **characterized in that** the hair cleansing agent has a pH in the range of from 4.3 to 5.0.

2. The hair cleansing agent according to claim 1, **characterized in that** it has a pH in the range of from 4.4 to 5.0, preferably from 4.5 to 4.9 and in particular from 4.5 to 4.8.

3. The hair cleansing agent according to one of the preceding claims, **characterized in that** the anionic surfactant is selected from ether carboxylic acids of formula R-O-(CH₂-CH₂O)ₓ-CH₂-COOH, in which R is a linear or branched, saturated or unsaturated alkyl group having 8 to 30 C atoms and x = 0 or 1 to 16, acyl isethionates having 8 to 24 C atoms in the acyl group, sulfosuccinic acid mono- and/or dialkyl esters having 8 to 24 C atoms in the alkyl group and sulfosuccinic acid monoalkyl polyoxyethyl esters having 8 to 24 C atoms in the alkyl group and 1 to 6 oxyethyl groups and from alkyl sulfate salts and/or alkyl polyglycol ether sulfate salts of formula R-(OCH₂-CH₂)ₓ-OSO₃⁻X⁺, in which R preferably is a linear or branched, saturated or unsaturated alkyl group having 8 to 30 C atoms, x is the number 0 or 1 to 12 and X⁺ is an alkali ion, an alkaline earth ion, an ammonium ion or an alkanolamine ion.

4. The hair cleansing agent according to one of the preceding claims, **characterized in that** it contains (based on the total weight thereof) 6 to 12 wt.%, preferably 7 to 11 wt.% and in particular 8 to 10 wt.% of at least one anionic alkyl ether sulfate salt of formula R-(OCH₂-CH₂)ₓ-OSO₃⁻ X⁺, in which R represents a straight-chain or branched alkyl functional group having 8 to 18 and in particular having 10 to 16 C atoms, x represents 1 to 6 and in particular 2 to 4 ethylene oxide units and X⁺ represents a sodium ion, a magnesium ion and/or a triethanolamine ion.

5. The hair cleansing agent according to one of the preceding claims, **characterized in that** the non-ionic surfactant is selected from C₈-C₃₀ fatty acid mono- and/or diesters of addition products of 1 to 30 mol ethylene oxide to glycerol, addition products of 2 to 50 mol ethylene oxide and/or 0 to 5 mol propylene oxide to linear and branched fatty alcohols having 8 to 30 C atoms, fatty acid alkanolamides of general formula 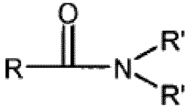 in which R is preferably a linear or branched, saturated or unsaturated alkyl or alkenyl functional group having 8 to 24 carbon atoms and the functional groups R' represent hydrogen or the group -(CH₂)ₙOH, in which n is the number 2 or 3, with the proviso that at least one of the functional groups R' represents the aforementioned functional group -(CH₂)ₙOH, and/or alkyl polyglucosides.

6. The hair cleansing agent according to one of the preceding claims, **characterized in that** it contains (based on the total weight thereof) 0.1 to 5 wt.%, preferably 0.2 to 3 wt.% and in particular 0.4 to 2 wt.% of at least one fatty acid alkanolamide of the aforementioned formula, preferably a fatty acid alkanolamide known by the INCI name cocamide MEA.

7. The hair cleansing agent according to one of the preceding claims, **characterized in that** the cationic conditioning polymer is selected from quaternized cellulose polymers, hydrophobically modified quaternized cellulose polymers, cationic guar derivatives, polymeric dimethyldiallylammonium salts and copolymers thereof with esters and/or amides of acrylic acid and methacrylic acid, copolymers of vinylpyrrolidone with quaternized derivatives of dialkylaminoalkyl acrylate and methacrylate and/or polyvinylpyrrolidone.

8. The hair cleansing agent according to one of the preceding claims, **characterized in that** it contains (based on the total weight thereof) 0.01 to 2 wt.%, preferably 0.1 to 1.5 wt.% and in particular 0.2 to 1 wt.% of at least one quaternized cellulose polymer, in particular a cationic polymer obtainable from the reaction of hydroxyethyl cellulose with trimethylammonium-substituted epoxides and known by the INCI name polyquaternium-10.

9. The hair cleansing agent according to one of the preceding claims, **characterized in that** it contains calcium chloride, calcium hydroxide, calcium sulfate and/or calcium oxide as the inorganic calcium salt d) (ii).

10. The hair cleansing agent according to one of the preceding claims, **characterized in that** it contains (based on the total weight thereof)
i. 0.01 to 3 wt.%, preferably 0.02 to 2.5 wt.% and in particular 0.05 to 2 wt.% calcium lactate or
ii. 0.1 to 5 wt.%, preferably 0.5 to 4 wt.% and in particular 1 to 3 wt.% lactic acid and 0.05 to 3 wt.%, preferably 0.1 to 2 wt.% and in particular 0.2 to 1 wt.% calcium hydroxide.

11. The hair cleansing agent according to one of the preceding claims, containing (based on the total weight thereof)
a) 6 to 12 wt.% of at least one anionic alkyl ether sulfate salt of formula R- (OCH₂-CH₂)ₓ-OSO₃⁻ X⁺, in which R represents a straight-chain or branched alkyl functional group having 8 to 18 and in particular having 10 to 16 C atoms, x represents 1 to 6 and in particular 2 to 4 ethylene oxide units and X⁺ represents a sodium cation, a magnesium cation and/or a triethanolamine cation,
b) 0.1 to 5 wt.% of at least one fatty acid alkanolamide of formula 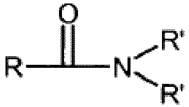 in which R is preferably a linear or branched, saturated or unsaturated alkyl or alkenyl functional group having 8 to 24 carbon atoms and the functional groups R' represent hydrogen or the group -(CH₂)ₙOH, in which n is the number 2 or 3, with the proviso that at least one of the functional groups R' represents the aforementioned functional group -(CH₂)ₙOH,
c) 0.01 to 2 wt.% of at least one quaternized cellulose polymer and
d)
(i) 0.01 to 3 wt.% calcium lactate or
(ii) 0.1 to 5 wt.% lactic acid and 0.05 to 3 wt.% calcium hydroxide,
**characterized in that** the hair cleansing agent has a pH in the range of from 4.3 to 5.0, preferably from 4.4 to 5.0, preferably from 4.5 to 4.9 and in particular from 4.5 to 4.8.

12. The hair cleansing agent according to one of the preceding claims, **characterized in that** it additionally contains (based on the total weight thereof) 0.01 to 5 wt.% of at least one silicone and/or of at least one vegetable oil.

13. The hair cleansing agent according to one of the preceding claims, **characterized in that** it additionally contains (based on the total weight thereof) 0.001 to 2 wt.% of at least one vitamin, vitamin derivative and/or vitamin salt and/or 0.001 to 5 wt.% of at least one - optionally cationized - protein hydrolyzate.

14. The hair cleansing agent according to one of the preceding claims, **characterized in that** it additionally contains (based on the total weight thereof) 0.01 to 0.5 wt.% of at least one UV filter, preferably a water-soluble UV filter.

15. The cosmetic use of a hair cleansing agent according to one of claims 1 to 14 for improving the untangling ability and combability of in particular damaged hair and for reducing the electrostatic charging ("fly-away") of hair and the development of frizzy or undesirably curly hair ("anti-frizz effect").

## Revendications

1. Composition nettoyante capillaire, contenant (par rapport à son poids total)
a) 6 à 12 % en poids d'au moins un tensioactif anionique,
b) 0,1 à 5 % en poids d'au moins un tensioactif non ionique,
c) 0,01 à 2 % en poids d'au moins un polymère de soin cationique et
d)
(i) 0,01 à 3 % en poids de lactate de calcium ou
(ii) 0,1 à 5 % en poids d'acide lactique et 0,05 à 3 % en poids d'au moins un sel de calcium inorganique,
**caractérisée en ce que** la composition nettoyante capillaire a un pH compris dans une plage allant de 4,3 à 5,0.

2. Composition nettoyante capillaire selon la revendication 1, **caractérisée en ce qu'**elle a un pH compris dans une plage allant de 4,4 à 5,0, de préférence de 4,5 à 4,9 et en particulier de 4,5 à 4,8.

3. Composition nettoyante capillaire selon l'une des revendications précédentes, **caractérisée en ce que** le tensioactif anionique est choisi parmi les acides éther carboxyliques de formule R-O-(CH₂-CH₂O)ₓ-CH₂-COOH, dans laquelle R est un groupe alkyle linéaire ou ramifié, saturé ou insaturé, ayant 8 à 30 atomes de carbone et x = 0 ou 1 à 16, des acyliséthionates ayant 8 à 24 atomes de carbone dans le groupe acyle, des mono et/ou dialkylesters sulfosucciniques ayant 8 à 24 atomes de carbone dans le groupe alkyle et des monoalkylpolyoxyéthylesters sulfosucciniques ayant 8 à 24 atomes de carbone dans le groupe alkyle et 1 à 6 groupes oxyéthyle ainsi que de sels de sulfate d'alkyle et/ou des sels de sulfate d'éther d'alkylpolyglycol de formule R-(OCH₂-CH₂)ₓ-OSO₃⁻X⁺, dans laquelle R correspond de préférence à un groupe alkyle linéaire ou ramifié, saturé ou insaturé ayant 8 à 30 atomes de carbone, x, au nombre 0 ou à un nombre compris entre 1 et 12, et X⁺, à un ion alcalin, alcalino-terreux, ammonium ou alcanolamine.

4. Composition nettoyante capillaire selon l'une des revendications précédentes, **caractérisée en ce qu'**elle représente (par rapport à son poids total) 6 à 12 % en poids, de préférence 7 à 11 % en poids et en particulier 8 à 10 % en poids d'au moins un sel de sulfate d'éther d'alkyle anionique de formule R-(OCH₂-CH₂)ₓ-OSO₃⁻ X⁺, dans laquelle R représente un radical alkyle à chaîne droite ou ramifié, ayant 8 à 18 et en particulier 10 à 16 atomes de carbone, x, 1 à 6 et en particulier 2 à 4 unités d'oxyde d'éthylène et X⁺ est un ion sodium, magnésium et/ou triéthanolamine.

5. Composition nettoyante capillaire selon l'une des revendications précédentes, **caractérisée en ce que** le tensioactif non ionique est choisi parmi les mono et/ou diesters d'acides gras en C₈-C₃₀ de produits d'addition de 1 à 30 moles d'oxyde d'éthylène sur du glycérol, de produits d'addition de 2 à 50 moles d'oxyde d'éthylène et/ou 0 à 5 moles d'oxyde de propylène sur des alcools gras linéaires et ramifiés ayant 8 à 30 atomes de carbone, des alcanolamides d'acides gras de formule générale 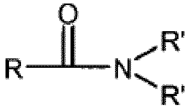 dans laquelle R représente de préférence un radical alkyle ou alcényle linéaire ou ramifié, saturé ou insaturé ayant de 8 à 24 atomes de carbone, et les radicaux R' représentent l'hydrogène ou le groupe -(CH₂)ₙOH, dans lequel n représente le nombre 2 ou le nombre 3, à condition qu'au moins l'un des radicaux R' représente le radical susmentionné -(CH₂)ₙOH , et/ou des alkylpolyglucosides.

6. Composition nettoyante capillaire selon l'une des revendications précédentes, **caractérisée en ce qu'**elle contient (par rapport à son poids total) 0,1 à 5 % en poids, de préférence 0,2 à 3 % en poids et en particulier 0,4 à 2 % en poids d'au moins un alcanolamide d'acide gras de formule susmentionnée, de préférence un alcanolamide d'acide gras connu sous le nom INCI Cocamide MEA.

7. Composition nettoyante capillaire selon l'une des revendications précédentes, **caractérisée en ce que** le polymère de soin cationique est choisi parmi les polymères de cellulose quaternisés, les polymères de cellulose quaternisés modifiés hydrophobiquement, les dérivés de guar cationiques, les sels polymères de diméthyldiallylammonium et leurs copolymères avec les esters et/ou les amides d'acide acrylique et méthacrylique, les copolymères de vinylpyrrolidone avec des dérivés quaternisés d'acrylate et de méthacrylate de dialkylaminoalkyle et/ou la polyvinylpyrrolidone.

8. Composition nettoyante capillaire selon l'une des revendications précédentes, **caractérisée en ce qu'**elle contient (par rapport à son poids total) 0,01 à 2 % en poids, de préférence 0,1 à 1,5 % en poids et en particulier 0,2 à 1 % en poids d'au moins un polymère de cellulose quaternisé, en particulier d'un polymère cationique obtenu par la réaction de l'hydroxyéthylcellulose avec des époxydes substitués par le triméthylammonium et connu sous le nom INCI Polyquaternium-10.

9. Composition nettoyante capillaire selon l'une des revendications précédentes, **caractérisée en ce qu'**elle contient, comme sel de calcium inorganique d), (ii) du chlorure de calcium, de l'hydroxyde de calcium, du sulfate de calcium et/ou de l'oxyde de calcium.

10. Composition nettoyante capillaire selon l'une des revendications précédentes, **caractérisée en ce qu'**elle contient (par rapport à son poids total)
i. 0,01 à 3 % en poids, de préférence de 0,02 à 2,5 % en poids et en particulier 0,05 à 2 % en poids de lactate de calcium ou
ii. 0,1 à 5 % en poids, de préférence 0,5 à 4 % en poids et en particulier 1 à 3 % en poids d'acide lactique, ainsi que 0,05 à 3 % en poids, de préférence 0,1 à 2 % en poids et en particulier 0,2 à 1 % en poids d'hydroxyde de calcium.

11. Composition nettoyante capillaire selon l'une des revendications précédentes, contenant (par rapport à son poids total)
a) 6 à 12 % en poids d'au moins un sel anionique de sulfate d'alkyléther de formule R-(OCH₂-CH₂)ₓ-OSO3⁻ X⁺, dans laquelle R représente un radical alkyle de chaîne linéaire ou ramifié, ayant 8 à 18 et en particulier 10 à 16 atomes de carbone, x, 1 à 6 et en particulier 2 à 4 unités d'oxyde d'éthylène, et X⁺, un cation sodium, magnésium et/ou triéthanolamine,
b) 0,1 à 5 % en poids d'au moins un alcanolamide d'acide gras de formule 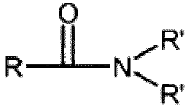 dans laquelle R représente de préférence un radical alkyle ou alcényle linéaire ou ramifié, saturé ou insaturé ayant de 8 à 24 atomes de carbone, et les radicaux R' représentent l'hydrogène ou le groupe -(CH₂)ₙOH , dans lequel n représente le nombre 2 ou le nombre 3, à condition qu'au moins un des radicaux R' représente le radical susmentionné -(CH₂)ₙOH ,
c) 0,01 à 2 % en poids d'au moins un polymère de cellulose quaternisé et
d)
(i) 0,01 à 3 % en poids de lactate de calcium ou
(ii) 0,1 à 5 % en poids d'acide lactique ainsi que 0,05 à 3 % en poids d'hydroxyde de calcium,
**caractérisée en ce que** la composition nettoyante capillaire a un pH compris dans une plage allant de 4,3 à 5,0, de préférence de 4,4 à 5,0, de préférence de 4,5 à 4,9 et en particulier de 4,5 à 4,8.

12. Composition nettoyante capillaire selon l'une des revendications précédentes, **caractérisée en ce qu'**elle contient en outre (par rapport à son poids total) 0,01 à 5 % en poids d'au moins une silicone et/ou d'au moins une huile végétale.

13. Composition nettoyante capillaire selon l'une des revendications précédentes, **caractérisée en ce qu'**elle contient en outre (par rapport à son poids total) 0,001 à 2 % en poids d'au moins une vitamine, un dérivé de vitamine et/ou un sel de vitamine et/ou 0,001 à 5 % en poids d'au moins un hydrolysat de protéines éventuellement cationisé.

14. Composition nettoyante capillaire selon l'une des revendications précédentes, **caractérisée en ce qu'**elle contient en outre (par rapport à son poids total) 0,01 à 0,5 % en poids d'au moins un filtre UV, de préférence d'un filtre UV hydrosoluble.

15. Utilisation cosmétique d'une composition nettoyante capillaire selon l'une des revendications 1 à 14, pour améliorer le démêlage et le coiffage, en particulier des cheveux abîmés, et pour réduire la charge électrostatique (« cheveux qui volent ») des cheveux et l'apparition de cheveux hirsutes ou bouclés de manière indésirable (« effet anti-frisottis »).
